# EUROPEAN PATENT SPECIFICATION

(11) **EP 3 868 393 B1**
(45) Date of publication and mention of the grant of the patent: **25.11.2026**
(21) Application number: 18937009.1
(22) Date of filing: 19.10.2018
(51) Int. Cl.: A61K 38/17, C07K 14/00, A61P 27/02, A61K 48/00, C07K 14/47

(54) **PHARMACEUTICAL COMPOSITION FOR TREATING RETINAL DYSTOPHY, COMPRISING NKX3.2 AND FRAGMENT THEREOF AS ACTIVE INGREDIENTS**
PHARMAZEUTISCHE ZUSAMMENSETZUNG ZUR BEHANDLUNG VON RETINALDYSTOPHY MIT NKX3.2 UND EINEM FRAGMENT DAVON ALS WIRKSTOFFE
COMPOSITION PHARMACEUTIQUE POUR LE TRAITEMENT DE DYSTROPHY RETINALE, COMPRENANT DU NKX3.2 ET UN FRAGMENT CORRESPONDANT À TITRE DE PRINCIPES ACTIFS

(43) Date of publication of application: 25.08.2021
(73) Proprietor: ICM Co., Ltd., Seoul 03722 (KR)
(72) Inventor: KIM, Dae-Won, Seoul 03719 (KR); YUM, Young-Na, Goyang-si Gyeonggi-do 10333 (KR); RYU, Heui-Young, Seoul 03719 (KR); JEONG, Da-Un, Seoul 03726 (KR); CHOI, Seung-Won, Suwon-si Gyeonggi-do 16697 (KR)
(74) Representative: Dehns
(86) International application number: PCT/KR2018/012428
(87) International publication number: WO 2020/080583

(56) References cited:
- WO-A2-2011/093647
- WO-A2-2012/097057
- WO-A2-2018/088813
- KR-A- 20110 087 462
- KR-A- 20180 118 552
- US-A1- 2004 242 482
- US-A1- 2010 216 660
- US-A1- 2018 236 035
- DATABASE Protein 5 August 2018 (2018-08-05), "homeobox protein Nkx-3.2 [Homo sapiens", XP055702277, retrieved from ncbi Database accession no. NP_001180.1
- ZAGOZEWSKI, J. L. ET AL.: "The role of homeobox genes in retinal development and disease", DEVELOPMENTAL BIOLOGY, vol. 393, 2014, pages 195 - 208, XP055613409, DOI: 10.1016/j.ydbio.2014.07.004

## Description

### Technical Field

The present invention relates to a pharmaceutical composition for preventing or treating retinal diseases, comprising Nkx3.2 and a fragment thereof as active ingredients.

### Background Art

As our society enters an aging society, interest in age-related diseases and treatments thereof is increasing. Among various senile diseases, macular degeneration, which is a representative senile eye disease, is a disease in which vision impairment is caused by degeneration in the macula located in the center of the retina inside the eye.

In macular degeneration, decreased vision or vision loss occurs due to decreased macular function caused by physical aging. This disease is a major cause of vision loss with age. Macular degeneration is a disease that usually occurs in the 50s and 60s, and may rarely occur in younger age groups. The disease is known to be caused by physical aging, cardiovascular disease, smoking, high level of blood cholesterol, environmental pollution, exposure to sunlight, or the like.

There are two types of macular degeneration: dry macular degeneration and wet macular degeneration. Dry macular degeneration occurs in a case where a type of aging-related deposit called drusen, is generated in the retina, or a lesion such as retinal pigment epithelial atrophy develops. About 90% of patients suffering from macular degeneration have dry macular degeneration. In dry macular degeneration, visual cells in the macula slowly undergo atrophy, and thus result in gradually decreased vision over time. Although dry macular degeneration does not cause severe vision loss, it may develop into a wet type. For wet macular degeneration, new blood vessels are abnormally formed in the choroid, and thus severe visual damage is likely to occur due to these blood vessels themselves, or bleeding, effusion, or the like therefrom. Wet macular degeneration may lead to blindness due to disc-like atrophy, severe bleeding, or the like within months to years after onset (Oh, M.J. & Lee, S.Y., 2012).

On the other hand, in a case where visual impairment begins due to macular degeneration, recovery to the previous vision cannot be achieved. Thus, it is important to detect and treat the macular degeneration at an early stage. Macular degeneration can be detected at an early stage in a case of using the Amsler Grid that is a self-assessment test for macular degeneration. As such, regarding macular degeneration, early detection and treatment thereof can minimize vision loss; however, there are currently no reliable treatments.

US 2018/236035 A1 relates to methods, compositions, and devices for treating an ocular disease, disorder or condition in a mammal.

WO 2011/093647 A2 relates to a composition for preventing or treating arthritis, a diagnosis kit for arthritis, a method for screening a composition for treating arthritis and a method for preventing or treating arthritis.

KR 2011/0087462 A relates to the development of therapeutic agents for degenerative arthritis using Nkx3.2 (NK3 homeobox 2) molecules.

US 2004/242482 A1 relates to methods for altering the fate of a cell, tissue or organ type by altering Notch pathway function in the cell.

### Disclosure of Invention

### Technical Problem

While studying therapeutic agents for macular degeneration, the present inventors have found that Nkx3.2 and/or a fragment thereof has an excellent effect on prevention or treatment of retinal dystrophies, such as inhibition of retinal pigment epithelial cell death and retinal degeneration, which are caused by oxidative stress, preservation of visual function, and inhibition of choroidal neovascularization and retinal edema. Based on this finding, the present inventors have completed the present invention.

An object of the present invention is to provide a pharmaceutical composition for use in preventing or treating retinal dystrophies, comprising, as an active ingredient, Nkx3.2 and/or a fragment thereof, wherein the Nkx3.2 or fragment thereof is a polypeptide consisting of the amino acid sequence of SEQ ID NO: 7 or 12 respectively.

### Solution to Problem

Aspects and embodiments of the present invention are defined in the claims. To achieve the above object, the present invention provides a pharmaceutical composition for use in preventing or treating retinal dystrophies, comprising, as an active ingredient, Nkx3.2 or a fragment thereof, wherein the Nkx3.2 or fragment thereof is a polypeptide consisting of the amino acid sequence of SEQ ID NO: 7 or 12 respectively.

### Effects of Invention

The Nkx3.2 and/or a fragment thereof comprised in the pharmaceutical composition for use according to the present invention inhibits retinal degeneration caused by oxidative stress and preserves visual function. In addition, the Nkx3.2 and/or a fragment thereof inhibits retinal pigment epithelial cell death caused by oxidative stress, and inhibits choroidal neovascularization and retinal edema. Therefore, a pharmaceutical composition comprising, as an active ingredient, the Nkx3.2 and/or a fragment thereof, wherein the Nkx3.2 or fragment thereof is a polypeptide consisting of the amino acid sequence of SEQ ID NO: 7 or 12 respectively, can be effectively used in prevention or treatment of retinal dystrophies.

### Brief Description of Drawings

FIG. 1 illustrates expression of Nkx3.2 protein in retinal pigment epithelial cells of C57BL/6 mice, detected through immunohistochemical staining. Here, OS is an abbreviation for outer segment.
FIG. 2 illustrates expression of Nkx3.2 protein in human retinal pigment epithelial cells, detected through immunohistochemical staining.
FIG. 3 illustrates expression of Nkx3.2 protein in human retinal pigment epithelial cells, detected through Western blot analysis. Here, WB abs is an abbreviation for Western blot antibodies.
FIG. 4 illustrates retinal pigment epithelial lesions in a retinal degeneration mouse model, the lesions having been caused by oxidative stress.
FIG. 5 illustrates expression of Nkx3.2 protein in a retinal degeneration mouse model, detected through immunohistochemical staining.
FIG. 6 illustrates expression of Nkx3.2 protein in a retinal degeneration mouse model, detected through Western blot analysis.
FIG. 7 illustrates a schematic diagram of a method for producing a mouse in which Nkx3.2 is overexpressed specifically in retinal pigment epithelial cells. The abbreviations illustrated in FIG. 7 have the following meanings: ci-Nkx3.2 = Cre-inducible Nkx3.2; ciTg-Nkx3.2 = Cre-inducible Nkx3.2 transgenic; and tpA = Transcription Stop.
FIG. 8 illustrates expression of Nkx3.2 protein in the retinal pigment epithelium of mice, in which Nkx3.2 is overexpressed specifically in retinal pigment epithelial cells, detected through immunohistochemical staining.
FIG. 9 illustrates degree of retinal degeneration depending on the expression level of Nkx3.2 protein, observed by retinal flat mount.
FIG. 10 illustrates degree of retinal degeneration in mice depending on the expression level of Nkx3.2 protein, observed through fundus image.
FIG. 11 illustrates degree of retinal degeneration in mice depending on the expression level of Nkx3.2 protein, observed through optical coherence tomography (OCT).
FIG. 12 illustrates degree of retinal degeneration in mice depending on the expression level of Nkx3.2 protein, observed through histopathology analysis.
FIG. 13 illustrates visual function preservation effects in mice depending on the expression level of Nkx3.2 protein, observed through electroretinography (ERG).
FIG. 14 illustrates cell viability, depending on H₂O₂ concentrations that induce oxidative stress, in human retinal pigment epithelial cells.
FIG. 15 illustrates cell death rate, depending on H₂O₂ concentrations that induce oxidative stress, in human retinal pigment epithelial cells.
FIG. 16 illustrates the expression level of Nkx3.2 protein and the expression level of cleaved PARP protein, a marker of cell death, depending on H₂O₂ concentrations that induce oxidative stress, in human retinal pigment epithelial cells, and they were detected through Western blot analysis.
FIG. 17 illustrates degree of cell death caused by oxidative stress, upon treatment with an Nkx3.2-expressing virus (Lenti-Nkx3.2), in human retinal pigment epithelial cells.
FIG. 18 illustrates degree of cell death caused by oxidative stress, upon treatment with a virus (sh-Nkx3.2) that inhibits Nkx3.2 expression, in human retinal pigment epithelial cells.
FIG. 19 illustrates ability of full-length Nkx3.2 and fragments thereof to inhibit retinal pigment epithelial cell death caused by oxidative stress, observed using a microplate reader device.
FIG. 20a illustrates transfection efficiency for full-length Nkx3.2 and fragments thereof, observed using an immunochemical technique.
FIGS. 20b and 20c illustrate results obtained by calculating the number of transfected Nkx3.2 proteins/number of DAPI-stained nuclei to obtain average values, and then representing the average values by a table (FIG. 20b) and a graph (FIG. 20c).
FIG. 21a illustrates images obtained by causing Nkx3.2-overexpressing mice to be irradiated with a laser so that choroidal neovascularization is induced, and then photographing retinal blood vessels by fundus angiography.
FIG. 21b illustrates a result obtained by comparing the difference in size of choroidal neovascular lesions between wild-type mice and Nkx3.2-overexpressing mice, based on the images photographed by fundus angiography.
FIG. 22a illustrates images obtained by causing Nkx3.2-overexpressing mice to be irradiated with a laser so that choroidal neovascularization is induced, and then photographing a retinal region by optical coherence tomography.
FIG. 22b illustrates a result obtained by comparing the difference in retinal edema between wild-type mice and Nkx3.2-overexpressing mice, based on the images photographed by optical coherence tomography.

### Best Mode for Carrying out the Invention

Hereinafter, the present invention will be described in detail.

In an aspect of the present invention, a pharmaceutical composition for use in preventing or treating retinal dystrophies, comprising, as an active ingredient, Nkx3.2 or a fragment thereof, wherein the Nkx3.2 or fragment thereof is a polypeptide consisting of the amino acid sequence of SEQ ID NO: 7 or 12 respectively, is provided.

The term "Nkx3.2", as used herein, is also called NK3 homeobox 2 and is one of the proteins belonging to the NK-2 homeobox family. Nkx3.2 plays an important role in skeletal development and organ differentiation. In particular, Nkx3.2 is known to promote chondrogenic differentiation, delay chondrocyte hypertrophy, and inhibit chondrocyte cell death. The Nkx3.2 may be a human-derived protein. Here, the Nkx3.2 protein consists of the amino acid sequence of SEQ ID NO: 7, which may be encoded by the nucleotide sequence of SEQ ID NO: 8.

The Nkx3.2 protein may be extracted from natural sources or may be prepared by synthesis (Merrifield, J. Amer. Chem. Soc. 85:2149-2156, 1963) or by a DNA sequence-based recombinant method (Sambrook, J. et al., 2001. Molecular Cloning. A Laboratory Manual, 3rd ed. Cold Spring Harbor Press).

The term "Nkx3.2 fragment", as used herein, is a polypeptide consisting of the amino acid sequence of SEQ ID NO: 12.

The polypeptide is a fragment of the Nkx3.2 protein and does not exist *in vivo.* However, the polypeptide is not easily degraded *in vivo* while having the same activity as the wild-type Nkx3.2 protein. Thus, the polypeptide exists for a longer time in the body than the wild-type Nkx3.2 protein, thereby exhibiting superior activity.

To evaluate the ability of the full-length Nkx3.2 and fragments thereof to inhibit retinal pigment epithelial cell death caused by oxidative stress, a DNA plasmid for each of the full-length Nkx3.2 and fragments thereof was transfected into a retinal pigment epithelial cell line, and oxidative stress was induced therein. As a result, it was identified that all of the full-length Nkx3.2 and fragments thereof inhibited retinal pigment epithelial cell death.

In another aspect of the present invention, there is provided a pharmaceutical composition for use in preventing or treating retinal dystrophies, comprising, as an active ingredient, a recombinant virus that contains a polynucleotide encoding Nkx3.2 or a fragment thereof, wherein the Nkx3.2 or fragment thereof is a polypeptide consisting of the amino acid sequence of SEQ ID NO: 7 or 12 respectively.

The virus may be any one selected from the group consisting of adenovirus, adeno-associated virus (AAV), retrovirus, lentivirus, herpes simplex virus, and vaccinia virus. Specifically, the virus may be adeno-associated virus (AAV). The adeno-associated virus is not limited to a specific serotype, and may preferably be any one of AAV1 to AAV9.

The adeno-associated virus (AAV) is suitable as a gene delivery system of the present invention because it is capable of infecting non-dividing cells and has an ability to infect various types of cells. Details on construction and use of AAV vectors are specifically disclosed in U.S. Patent Nos. 5,139,941 and 4,797,368.

Typically, the AAV virus may be produced by co-transfection of a plasmid comprising a gene sequence of interest flanked by two AAV terminal repeats and an expression plasmid comprising a wild-type AAV coding sequence without terminal repeats.

The term "retinal dystrophy", as used herein, refers to a disease caused by damage that has occurred to the retina for reasons such as aging and disease. The retinal dystrophy may be any one selected from the group consisting of macular degeneration, diabetic retinopathy, choroidal neovascularization, and retinal edema.

The macular degeneration is a disease in which the macular function decreases as aging progresses, so that vision decreases or is lost. In a case where vision starts to decrease due to this disease, recovery to the original vision cannot be achieved. The disease is called age-related macular degeneration and is a leading cause of vision loss in old age. The macular degeneration is divided into two types: dry macular degeneration and wet macular degeneration. About 90% of patients suffering from macular degeneration have dry macular degeneration, and this dry macular degeneration occurs in a case where age-related deposits accumulate under the retina or lesions such as retinal pigment epithelial atrophy develop. As visual cells in the macula are slowly destroyed, the macular function decreases, and the central vision decreases over time. For wet macular degeneration, symptoms occur due to formation of abnormally many new blood vessels in the choroid that make up the lower layer of the macula. Wet macular degeneration progresses faster than dry macular degeneration, which may lead to a sharp drop in vision and lead to blindness within 2 months to 3 years.

Diabetic retinopathy is a complication that occurs in the retina of the eye due to peripheral circulatory disorder caused by diabetes. The disease may initially have no symptoms and may exhibit symptoms of decreased vision as its invasion into the macula occurs. Diabetic retinopathy may be divided into simple retinopathy, pre-proliferative retinopathy, and proliferative retinopathy, depending on the progression stage.

Choroidal neovascularization is a disease in which the choroid is damaged due to formation of abnormal blood vessels, so that visual impairment occurs. The choroidal neovascularization is one of the leading causes of irreversible vision loss worldwide. For the choroidal neovascularization, despite various therapeutic attempts, prognosis of vision is poor for most patients.

Retinal edema means that the retina is swollen. As a degeneration or abnormality occurs in fine blood vessels such as capillaries in the retina or macula for various reasons, bleeding may occur, resulting in retinal edema. In a case where edema occurs in the retina, various symptoms may occur, including decreased vision.

In the pharmaceutical composition comprising Nkx3.2 or a fragment thereof, wherein the Nkx3.2 or fragment thereof is a polypeptide consisting of the amino acid sequence of SEQ ID NO: 7 or 12 respectively, for use according to the present invention, the polypeptide may be contained in an amount of 10% to 95% by weight with respect to the total weight of the pharmaceutical composition. Also, in addition to the active ingredient, the pharmaceutical composition for use of the present invention may further comprise one or more active ingredients that exhibit the same or similar function. In addition to the above-described active ingredients, the pharmaceutical composition for use according to the present invention may further comprise one or more pharmaceutically acceptable carriers for administration.

In addition, a dose of the pharmaceutical composition for use according to the present invention, comprising, as an active ingredient, a recombinant virus that contains a polynucleotide encoding Nkx3.2 or a fragment thereof, wherein the Nkx3.2 or fragment thereof is a polypeptide consisting of the amino acid sequence of SEQ ID NO: 7 or 12 respectively, may vary depending on various factors including type of disease, severity of disease, types and amounts of active ingredients and other ingredients contained in the pharmaceutical composition, type of formulation and the patient's age, weight, general health status, gender and diet, time of administration, route of administration, duration of treatment, and simultaneously used drugs.

The recombinant virus contained in the pharmaceutical composition for use according to the present invention may be administered in an amount of 1.0 × 10⁵ to 1.0 × 10¹⁵ viral genomes per day on an adult basis so that the pharmaceutical composition exhibits a desirable effect. Specifically, in a dose of the pharmaceutical composition for use of the present invention, the virus may be administered in an amount of 1.0 × 10⁵ to 1.0 × 10¹⁵, 1.0 × 10⁷ to 1.0 × 10¹³, 1.0 × 10⁸ to 1.0 × 10¹², or 1.0 × 10⁹ to 1.0 × 10¹⁰ per day on an adult basis.

A dose of the polypeptide that is Nkx3.2 or a fragment thereof consisting of the amino acid of SEQ ID NO: 7 or 12 respectively; or the recombinant virus that contains a polynucleotide encoding Nkx3.2 or a fragment thereof, wherein the Nkx3.2 or fragment thereof is a polypeptide consisting of the amino acid sequence of SEQ ID NO: 7 or 12 respectively, comprised within the pharmaceutical composition for use according to the present invention, may vary depending on various factors including type of disease, severity of disease, types and amounts of active ingredients and other ingredients contained in the pharmaceutical composition, type of formulation and the patient's age, weight, general health status, gender and diet, time of administration, route of administration, duration of treatment, and simultaneously used drugs.

In the pharmaceutical composition for use in preventing or treating retinal dystrophies, comprising, as an active ingredient, Nkx3.2 or a fragment thereof, wherein the Nkx3.2 or fragment thereof is a polypeptide consisting of the amino acid sequence of SEQ ID NO: 7 or 12 respectively, according to the present invention, the polypeptide that is Nkx3.2 or a fragment thereof may be contained in an effective amount of 0.0001 to 100 mg/kg so that the pharmaceutical composition exhibits a desirable effect. Here, the administration may be performed once or several times a day.

In addition, in the pharmaceutical composition for use in preventing or treating retinal dystrophies, comprising, as an active ingredient, a recombinant virus that contains a polynucleotide encoding Nkx3.2 or a fragment thereof, wherein the Nkx3.2 or fragment thereof is a polypeptide consisting of the amino acid sequence of SEQ ID NO: 7 or 12 respectively, according to the present invention, the recombinant virus may be administered in an amount of 1.0 × 10⁵ to 1.0 × 10¹⁵ viral genomes per day on an adult basis so that the pharmaceutical composition exhibits a desirable effect. Specifically, in the pharmaceutical composition for use of the present invention, the virus may be administered in an amount of 1.0 × 10⁵ to 1.0 × 10¹⁵, 1.0 × 10⁷ to 1.0 × 10¹³, 1.0 × 10⁸ to 1.0 × 10¹², or 1.0 × 10⁹ to 1.0 × 10¹⁰ per day on an adult basis.

In addition, the pharmaceutical composition for use of the present invention may be administered to an individual in need thereof by various methods known in the art. The individual may be a mammal, specifically a human. The route of administration may be appropriately selected by a person skilled in the art in consideration of administration method, body fluid volume, viscosity, and the like. Specifically, the administration may be performed via any one selected from the group consisting of intravenous, intraarterial, intraperitoneal, intramuscular, intrasternal, transdermal, intranasal, inhalation, topical, rectal, oral, intraocular, and intradermal routes.

### Embodiment of the Invention

Hereinafter, the present invention will be described in detail by way of the following examples. However, the following examples are only to illustrate the present invention, and the present invention is not limited thereto.

### Preparation Example 1. Preparation of experimental ocular tissue

C57BL/6 mice were purchased and kept under a specific sterile condition in an approved animal facility. According to a predetermined experimental protocol, the eyes of normal C57BL/6 mice were excised and the tissue was separated therefrom.

In addition, human ocular tissue was purchased and kept under a specific sterile condition in an approved facility.

### Experimental Example 1. Identification of expression of Nkx3.2 in retinal pigment epithelial cells

Since expression of the Nkx3.2 protein in the ocular retinal pigment epithelium had never been reported, verification therefor was made using immunohistochemistry (IHC) staining and Western blot analysis.

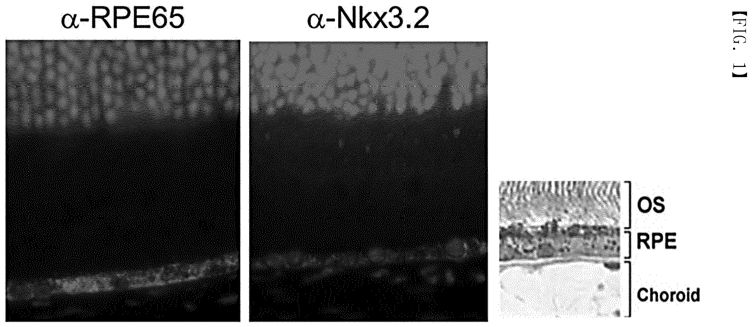

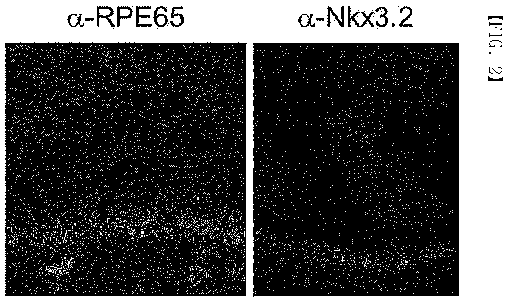

In the C57BL/6 mouse ocular tissue and the human ocular tissue prepared in Preparation Example 1, expression of the Nkx3.2 protein was identified using immunohistochemistry staining. The results are illustrated in FIGS. 1 and 2.

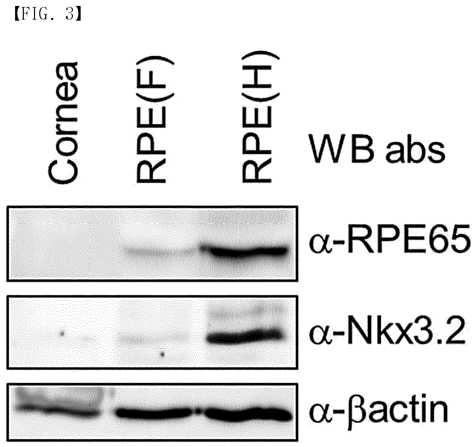

In addition, the cornea and the retinal pigment epithelium (RPE) were separated from the human ocular tissue prepared in Preparation Example 1, and the proteins were extracted therefrom. Expression of the Nkx3.2 protein was identified through Western blot analysis. The results are illustrated in FIG. 3.

As illustrated in FIGS. 1 and 2, expression of the Nkx3.2 protein was identified in the C57BL/6 mouse and human ocular tissues along with RPE65 protein that was specifically expressed in retinal pigment epithelial cells. In addition, as illustrated in FIG. 3, it was identified that the Nkx3.2 protein was expressed higher in the hind (H) part of the eye where RPE65 was highly expressed than in the frontal (F) part of the eye.

### Experimental Example 2. Identification of expression of Nkx3.2 in retinal degeneration model induced by oxidative stress

Immunohistochemistry (IHC) staining and Western blot analysis were used to identify expression of Nkx3.2 protein in a retinal degeneration model induced by oxidative stress.

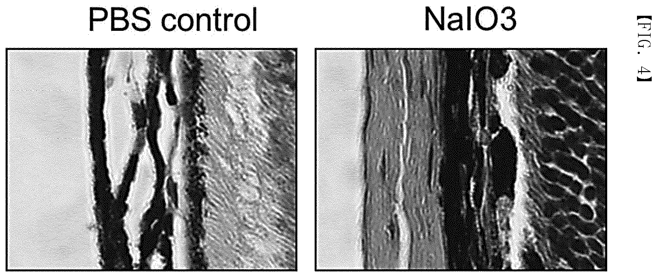

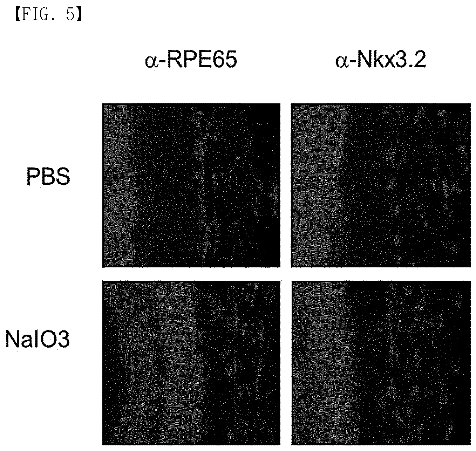

The retinal degeneration mouse model was constructed by inducing oxidative stress, in the eyes of the C57BL/6 mice prepared in Preparation Example 1, using sodium iodate (NaIO₃). Then, lesions in the retinal degeneration mouse model were observed using a microscope. In addition, expression of the Nkx3.2 protein in the retinal degeneration mouse model was identified using immunohistochemistry staining. The results are illustrated in FIGS. 4 and 5.

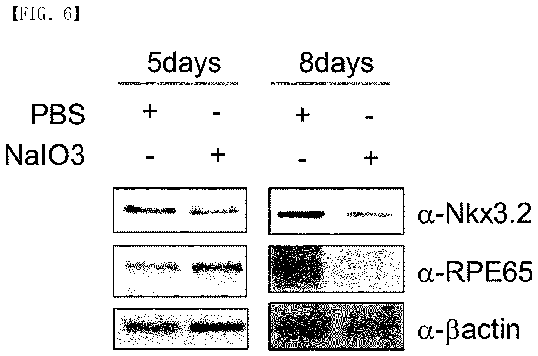

In addition, proteins were extracted from the tissue of the retinal degeneration mouse model and subjected to Western blot analysis, to identify expression of the Nkx3.2 protein. The results are illustrated in FIG. 6.

As illustrated in FIG. 4, the retinal pigment epithelial lesion was identified in the retinal degeneration mouse model constructed by inducing oxidative stress using NaIO₃. In addition, as illustrated in FIG. 5, it was identified that the Nkx3.2 protein was decreased in the retinal degeneration mouse model in which the retinal pigment epithelial lesion was identified.

In addition, as illustrated in FIG. 6, it was identified that expression of the Nkx3.2 protein was remarkably decreased in the retinal degeneration model induced by oxidative stress, and such a decrease was an initial change preceding a decrease in RPE65 which is a marker of the retinal pigment epithelium.

### Experimental Example 3. Generation of mice overexpressing Nkx3.2 in retinal pigment epithelial cells

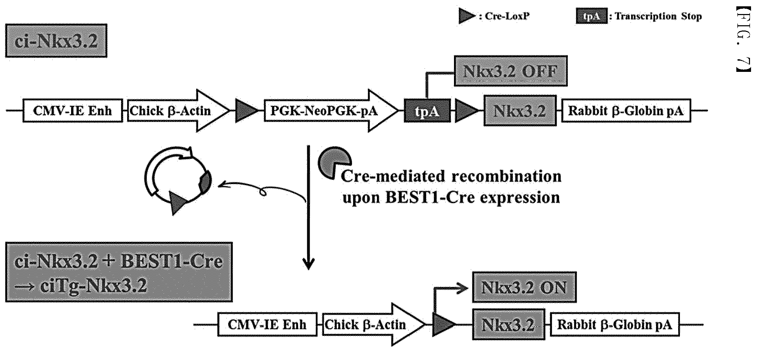

In a previous paper, the present research team reported Cre-dependent Nkx3.2-overexpressing mice (Jeong, Da-Un, Je-Yong Choi, and Dae-Won Kim. "Cartilage-Specific and Cre-Dependent Nkx3. 2 Overexpression In Vivo Causes Skeletal Dwarfism by Delaying Cartilage Hypertrophy." Journal of cellular physiology 232.1 (2017): 78-90). Based on this, BEST1-Cre mice, in which Cre is expressed specifically in retinal pigment epithelial cells, were engineered such that overexpression of Nkx3.2 by Cre gene recombinase occurs only in retinal pigment epithelial cells. Production strategy for the corresponding mice is schematically illustrated in FIG. 7.

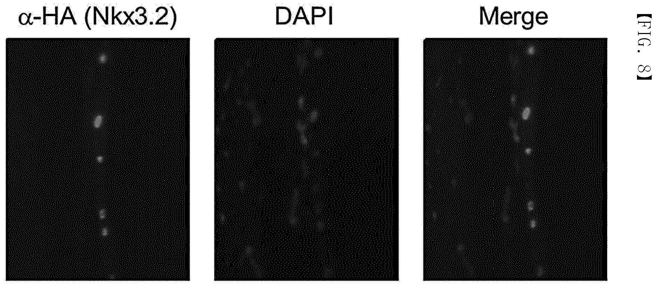

In addition, expression of Nkx3.2 in the retinal pigment epithelial cells of the produced Nkx3.2-overexpressing mice was identified through immunohistochemistry staining, and the results are illustrated in FIG. 8.

As illustrated in FIG. 8, for the mice produced such that overexpression of Nkx3.2 by Cre gene recombinase occurs only in retinal pigment epithelial cells, overexpression of the Nkx3.2 protein was verified in the retinal pigment epithelial cells.

### Experimental Example 4. Identification of retinal pigment epithelial cell inhibition effect caused by overexpression of Nkx3.2

For the mice produced in Experimental Example 3 in which Nkx3.2 was overexpressed specifically in retinal pigment epithelial cells, retinal degeneration was induced by oxidative stress generated using NaIO₃.

### Experimental Example 4.1. Retinal flat mount

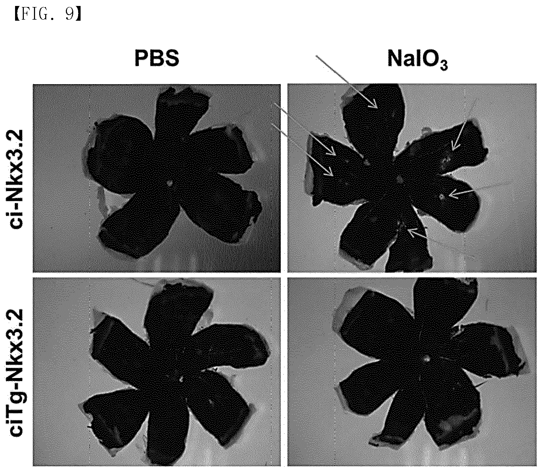

For the mice in which retinal degeneration was induced by oxidative stress, the hemispherical retina was flat-mounted to identify retinal degenerative lesions. The results are illustrated in FIG. 9.

As illustrated in FIG. 9, in the Nkx3.2 protein-non-overexpressing (ci-Nkx3.2) mice, retinal degeneration caused by oxidative stress was clearly observed. On the other hand, in the Nkx3.2-overexpressing (ciTg-Nkx3.2) mice, retinal degeneration caused by oxidative stress was not observed.

### Experimental Example 4.2. Fundus image

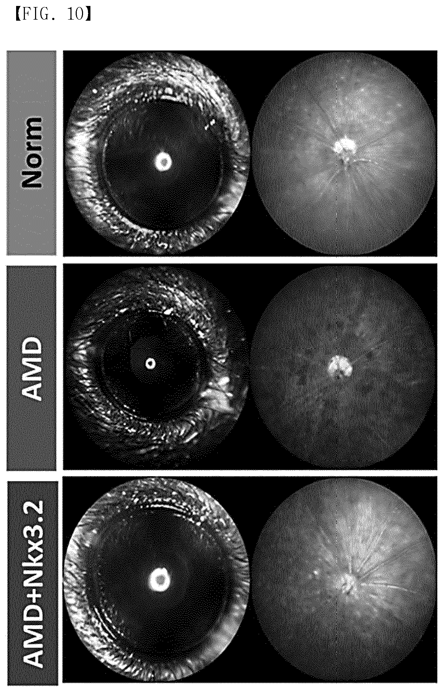

The ocular retinal structure of the mice, in which retinal degeneration was induced by oxidative stress, was analyzed by fundus image. The results are illustrated in FIG. 10.

As illustrated in FIG. 10, the normal control group (Norm) exhibited a uniform and even form of retinal curve around the optic nerve and exhibited normal retinal reflection. On the other hand, the retinal degeneration group of Nkx3.2-non-overexpressing mice (AMD) exhibited split retinal pigment epithelium and exhibited low reflection pattern. In contrast, the retinal degeneration group of Nkx3.2-overexpressing mice (AMD+Nkx3.2) exhibited a similar retinal structure to that of the normal control group.

### Experimental Example 4.3. Optical coherence tomography

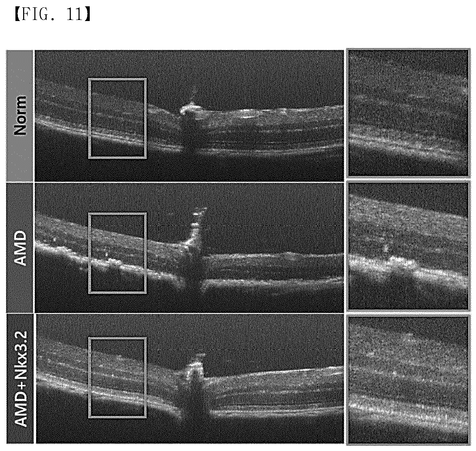

The ocular retinal structure of mice, in which retinal degeneration was induced by oxidative stress, was analyzed using optical coherence tomography. The results are illustrated in FIG. 11.

As illustrated in FIG. 11, the normal control group (Norm) well exhibited a multilayered structure of the retina, whereas the retinal degeneration group of Nkx3.2-non-overexpressing mice (AMD) had severe retinal degeneration and exhibited a severely damaged multilayered structure of the retina. In contrast, the retinal degeneration group of Nkx3.2-overexpressing mice (AMD+Nkx3.2) exhibited a similar retinal structure to that of the normal control group.

### Experimental Example 4.4. Histopathological analysis

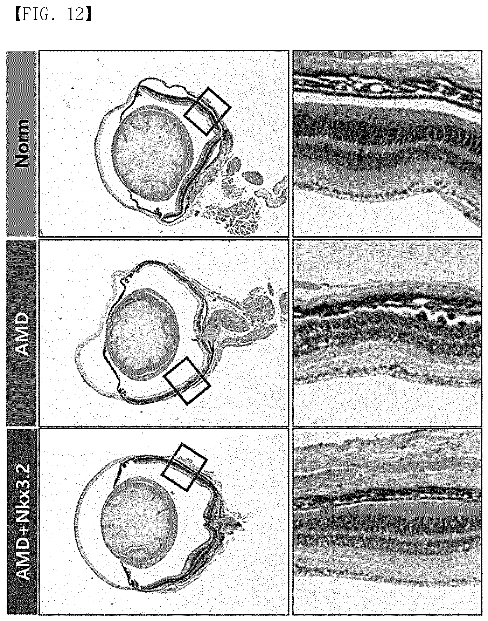

The ocular retinal structure of the mice, in which retinal degeneration was induced by oxidative stress, was histologically analyzed. The results are illustrated in FIG. 12.

As illustrated in FIG. 12, the normal control group (Norm) exhibited a normal structure in the retina and a normal membrane structure in the retinal pigment epithelium. On the other hand, the retinal degeneration group of Nkx3.2-non-overexpressing mice (AMD) exhibited the retinal pigment epithelium that was severely damaged and lost. In contrast, the retinal degeneration group of Nkx3.2-overexpressing mice (AMD+Nkx3.2) exhibited similar retinal structure and retinal pigment epithelial pattern to those of the normal control group.

### Experimental Example 5. Identification of visual function preservation effect caused by overexpression of Nkx3.2

A visual function preservation effect caused by overexpression of Nkx3.2 was identified through electroretinography (ERG).

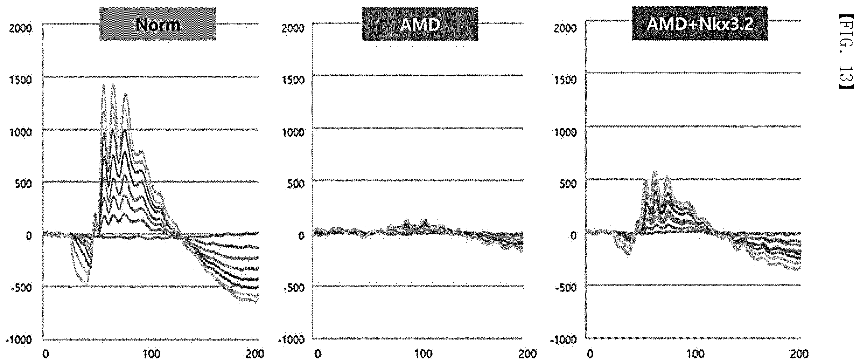

For the mice in which retinal degeneration was induced by oxidative stress in Experimental Example 4 and in which Nkx3.2 was overexpressed specifically in retinal pigment epithelial cells, their visual function was analyzed through ERG. The results are illustrated in FIG. 13.

As illustrated in FIG. 13, the normal control group (Normal) exhibited a normal retinal potential change in response to an external light stimulus applied to the retina. On the other hand, the retinal degeneration group of Nkx3.2-non-overexpressing mice (AMD) had complete disappearance of a retinal potential change occurring in response to an external light stimulus. In contrast, the retinal degeneration group of Nkx3.2-overexpressing mice (AMD+Nkx3.2) exhibited a similar retinal potential change to that of the normal control group.

### Experimental Example 6. Identification of cell viability, cell death rate, and expression of Nkx3.2 protein, depending on H₂O₂ concentrations that induce oxidative stress

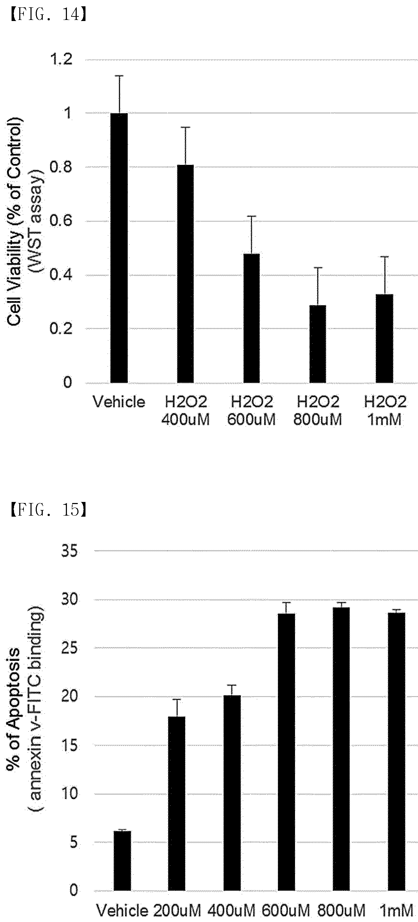

Human retinal pigment epithelial cells were treated with 400 µM, 600 µM, 800 µM, and 1 mM hydrogen peroxide (H₂O₂), respectively, to induce oxidative stress. Thereafter, cell viability, depending on H₂O₂ concentrations that induce oxidative stress, was measured using a water-soluble tetrazolium salt assay (WST). The results are illustrated in FIG. 14.

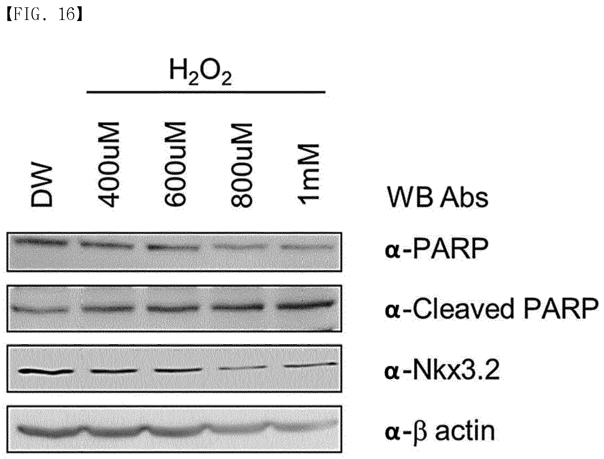

In addition, a cell death rate in the human retinal pigment epithelial cells, in which oxidative stress was induced by treatment with the above-mentioned respective H₂O₂ concentrations, was measured using an annexin V-FITC apoptosis kit (Sigma-Aldrich). In addition, changes in expression level of the Nkx3.2 protein, depending on increased cell death rate, were measured through Western blot analysis. The results are illustrated in FIGS. 15 and 16.

As illustrated in FIG. 14, the cell viability decreased as the H₂O₂ concentration increased. In addition, as illustrated in FIG. 15, the cell death rate increased as the H₂O₂ concentration increased. As illustrated in FIG. 16, expression of cleaved PARP protein, which is an apoptotic marker, increased as the H₂O₂ concentration increased. That is, it was verified at the protein level that increased expression of the cleaved PARP protein, which is an apoptotic marker, caused cell death. In addition, it was identified that expression of the Nkx3.2 protein decreased as expression of the cleaved PARP protein increased.

### Experimental Example 7. Measurement of cell death rate after administration of Nkx3.2 protein-expressing virus

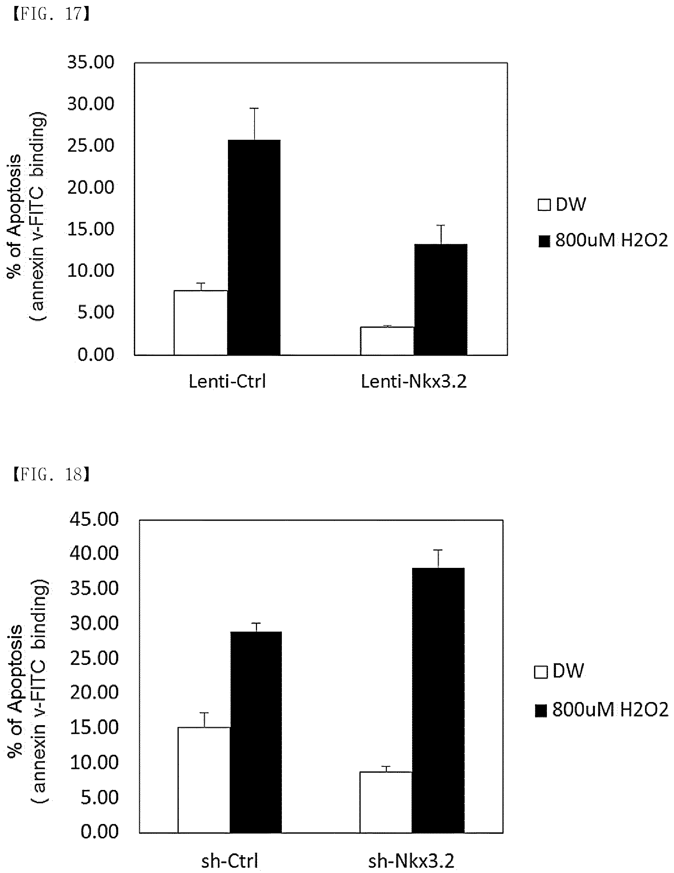

Human retinal pigment epithelial cells were treated with 800 µM H₂O₂, to induce oxidative stress. The oxidative stress-induced cells were divided into two groups. One group was administered the Nkx3.2-expressing virus (Lenti-Nkx3.2), and the other group was administered the virus (sh-Nkx3.2) with suppressed expression of Nkx3.2. Thereafter, a cell death rate caused by oxidative stress in the human retinal pigment epithelial cells, depending on the administration of each virus, was measured using the annexin V-FITC apoptosis kit. The results are illustrated in FIGS. 17 and 18.

As illustrated in FIG. 17, in a case where the human retinal pigment epithelial cells were administered the Nkx3.2-expressing virus (Lenti-Nkx3.2), the cell death rate caused by H₂O₂ was decreased as compared with the control virus (Lenti-Ctrl)-administered group. In addition, as illustrated in FIG. 18, in a case where the human retinal pigment epithelial cells were administered the virus (sh-Nkx3.2) with suppressed expression of Nkx3.2, the cell death rate caused by H₂O₂ was increased as compared with the control virus (sh-Ctrl)-administered group.

### Experimental Example 8. Evaluation of ability of Nkx3.2 protein and fragments thereof to inhibit retinal pigment epithelial cell death caused by oxidative stress

### Experimental Example 8.1. Evaluation of ability of full-length Nkx3.2 protein and fragments thereof to inhibit retinal pigment epithelial cell death caused by oxidative stress.

To evaluate whether the full-length Nkx3.2 and fragments thereof have a function of inhibiting retinal pigment epithelial cell death caused by H₂O₂, which is an oxidative stress, ARPE19 cells were seeded in a 12-well plate (3E+4 cells/well) and transfection was carried out with 0.6 µg of a DNA plasmid encoding each of the following: the full-length hNkx3.2 (SEQ ID NO: 7), and fragments of hNkx3.2 which have, respectively, the amino acid sequence of SEQ ID NO: 9 (amino acids 1-320 of the full-length hNkx3.2 sequence) (not claimed), SEQ ID NO: 10 (amino acids 1-307 of the full-length hNkx3.2 sequence) (not claimed), SEQ ID NO: 12 (amino acids 99-333 of the full-length hNkx3.2 sequence), SEQ ID NO: 13 (amino acids 112-333 of the full-length hNkx3.2 sequence) (not claimed), SEQ ID NO: 20 (amino acids 112-320 of the full-length hNkx3.2 sequence) (not claimed), SEQ ID NO: 21 (amino acids 123-320 of the full-length hNkx3.2 sequence) (not claimed), SEQ ID NO: 23 (amino acids 150-320 of the full-length hNkx3.2 sequence) (not claimed), and SEQ ID NO: 28 (amino acids 165-310 of the full-length hNkx3.2 sequence) (not claimed).

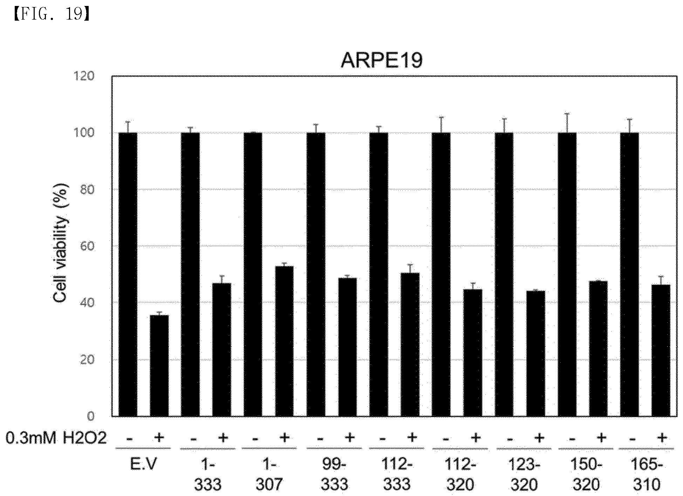

36 hours after transfection, reaction with 0.3 mM H₂O₂ was allowed to proceed in a CO₂ incubator at 37°C for 15 hours. 50 µl of water-soluble tetrazolium salt was added to each well, and then reaction was allowed to proceed in a CO₂ incubator at 37°C for 90 minutes. 150 ul of the supernatant was taken from each well and transferred to a 96-well plate. Absorbance was measured at a wavelength of 450 nm using a microplate reader device. The results are illustrated in FIG. 19.

As illustrated in FIG. 19, the Nkx3.2 fragments, including the full-length Nkx3.2, inhibited ARPE19 cell death caused by H₂O₂ from as little as 8% to as much as 17% as compared with the control group.

### Experimental Example 8.2. Evaluation of transfection efficiency for full-length Nkx3.2 and fragments thereof

Due to the fact that 100% efficiency does not appear given the characteristics of transfection, there is a possibility that in the cell viability analysis performed in the above experimental example, the ability of the full length Nkx3.2 and fragments thereof to inhibit retinal pigment epithelial cell death caused by H₂O₂ was undervalued. In this regard, the transfection efficiency for the full-length Nkx3.2 and fragments thereof was evaluated using an immunocytochemistry technique. First, AREP19 cells were seeded in a 12-well plate containing a slide glass with 2E+4 cells/well. Transfection was performed with 0.6 µg of DNA plasmid encoding each of the full-length hNkx3.2 or fragments thereof. After 48 hours, 2% paraformaldehyde was added to each well and reaction was allowed to proceed at room temperature for 15 minutes, so that the cells were fixed. Thereafter, 0.1% Triton X-100 solution in 0.1% sodium citrate was added to each well and reaction was allowed to proceed at room temperature for 30 minutes, so that the cells were made in a state where antibodies easily penetrate the cells. Then, washing with PBS was performed twice.

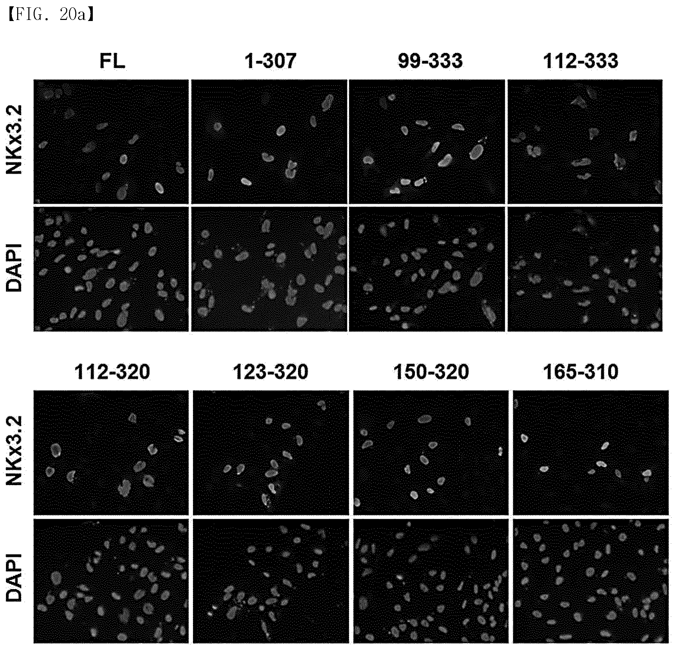

0.1% Triton X-100 solution in 5% goat serum was added to each well and reaction was allowed to proceed at room temperature for 30 minutes, so that nonspecific staining of antibodies was decreased. Then, washing with 0.05% Tween 20 in PBS (PBST) was performed twice. The primary antibody (Nkx3.2-rabbit) was diluted in a ratio of 1:500 and added thereto. Reaction was allowed to proceed 4°C for 16 hours and washing with PBST was performed 5 times. The secondary antibody (FITC-anti-rabbit) was diluted in a ratio of 1:300 and added thereto. Reaction was allowed to proceed at room temperature for 30 minutes and washing with PBST was performed 5 times. DAPI was diluted in a ratio of 1:500 and added thereto. Reaction was allowed to proceed at room temperature for 10 minutes and washing with PBST was performed twice. The slide glass was wiped off with 70% EtOH. Then, mounting was performed with an anti-fading mounting solution and drying was performed in a dark room for 24 hours. Then, each sample was photographed with an upright microscope. Each sample was photographed in its column section. The number of transfected Nkx3.2 proteins per DAPI staining was counted and the average value was calculated. The results are illustrated in FIGS. 20a to 20c.

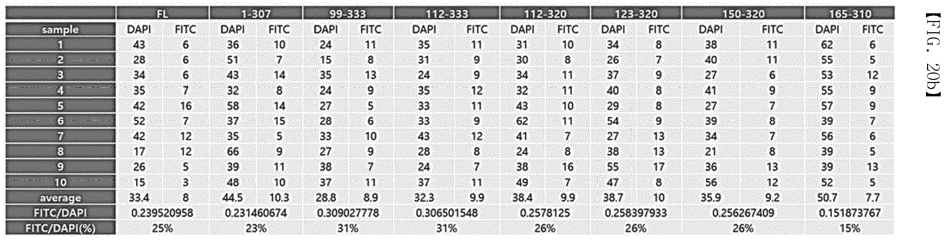

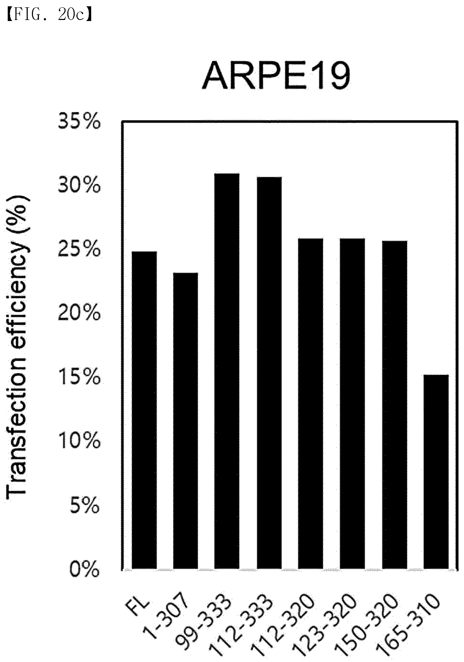

FIG. 20a illustrates the transfected Nkx3.2 and DAPI-stained nuclei, which were observed using an immunocytochemistry technique. As illustrated in FIGS. 20b and 20c, the transfection efficiency for the full-length Nkx3.2 and fragments thereof with respect to ARPE19 cells was between 15% and 31%.

### Experimental Example 8.3. Re-evaluation of ability of full-length Nkx3.2 and fragments thereof to inhibit retinal pigment epithelial cell death caused by oxidative stress, depending on their transfection efficiency

In consideration of the transfection efficiency for the full-length Nkx3.2 and fragments thereof, re-evaluation was performed on whether they could inhibit ARPE19 cell death caused by H₂O₂. The results are shown in Table 1 below.

**[Table 1]**

| **Construct's SEQ ID NO** | **Measured cell viability enhancement (%)** | **Transfection efficiency for each construct (%)** | **Adjusted cell viability enhancement (%)** |
|---|---|---|---|
| SEQ ID NO: 7 (Nkx3.2) | 11 | 25 | 44.0 |
| SEQ ID NO: 10 (amino acids 1-307 of full-length Nkx3.2 sequence) (not claimed) | 17 | 23 | 73.9 |
| SEQ ID NO: 12 (amino acids 99-333 of full-length Nkx3.2 sequence) | 13 | 31 | 41.9 |
| SEQ ID NO: 13 (amino acids 112-333 of full-length Nkx3.2 sequence) (not claimed) | 14 | 31 | 45.2 |
| SEQ ID NO: 20 (amino acids 112-320 of full-length Nkx3.2 sequence) (not claimed) | 9 | 26 | 34.6 |
| SEQ ID NO: 21 (amino acids 123-320 of full-length Nkx3.2 sequence) (not claimed) | 8 | 26 | 30.8 |
| SEQ ID NO: 23 (amino acids 150-320 of full-length Nkx3.2 sequence) (not claimed) | 12 | 16 | 75.0 |
| SEQ ID NO: 28 (amino acids 165-310 of full-length Nkx3.2 sequence) (not claimed) | 10 | 15 | 50.0 |

As shown in Table 1, the Nkx3.2 fragments as well as the full-length Nkx3.2, inhibited ARPE19 cell death caused by H₂O₂ from as little as 30% to as much as 75%, as compared with the control group.

### Experimental Example 9. Identification of choroidal neovascularization inhibition effect caused by overexpression of Nkx3.2

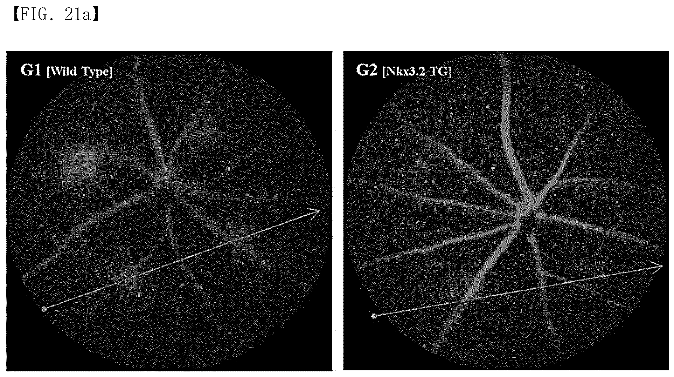

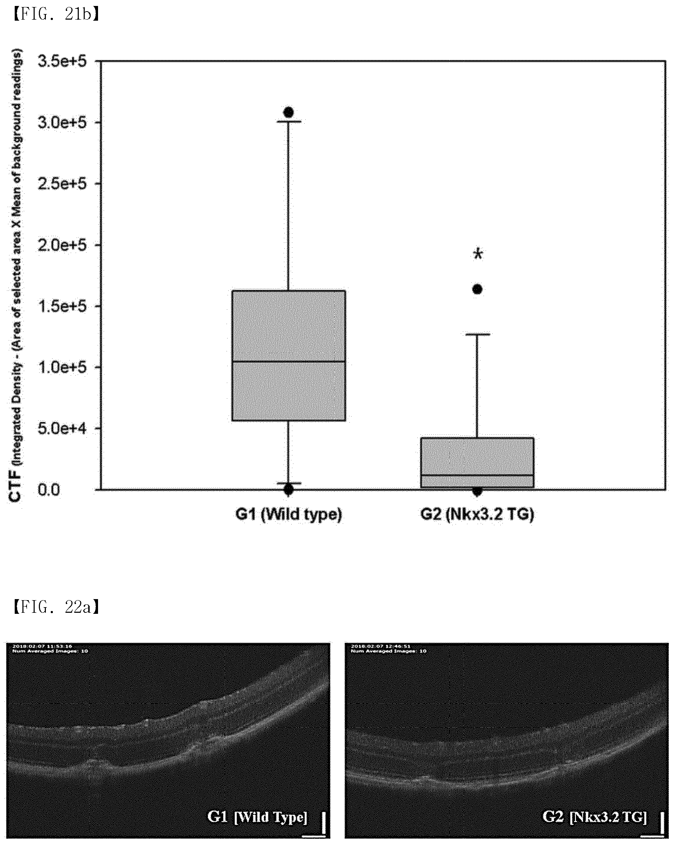

The mice, in which Nkx3.2 was overexpressed specifically in retinal pigment epithelial cells, were irradiated with a laser to induce choroidal neovascularization (CNV). After 9 days, the mice were intraperitoneally injected with a contrast agent, and fundus fluorescence angiography (FAA) was performed on retinal blood vessels. Based on the images obtained from the fundus fluorescence angiography, CTF of the choroidal neovascular lesion was measured. A comparison was made, in terms of difference in size of the choroidal neovascular lesion, between the wild-type mice and the Nkx3.2-overexpressing mice. The results are illustrated in FIGS. 21A and 21B.

As illustrated in FIGS. 21A and 21B, a significantly decreased CTF value (29414.8±43200.47) for the choroidal neovascular lesion was observed in Nkx3.2-overexpressing mice (G2) as compared with that (124942.3±91743.74) in the wild-type mice (G1) (P<0.05). From these results, it was found that Nkx3.2 played an important role in inhibiting choroidal neovascularization.

### Experimental Example 10. Identification of retinal edema inhibition effect caused by overexpression of Nkx3.2

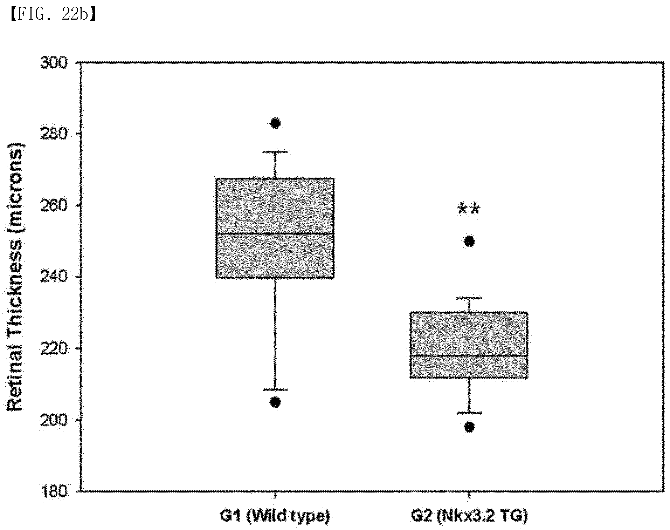

The mice, in which Nkx3.2 was overexpressed specifically in retinal pigment epithelial cells, were irradiated with a laser to induce choroidal neovascularization. Then, on Day 9, optical coherence tomography was performed on the choroidal neovascularization-induced retinal region. Based on the images obtained from the optical coherence tomography, a comparison was made, in terms of difference in retinal edema pattern caused by the choroidal neovascular lesion, between the wild-type mice and the Nkx3.2-overexpressing mice. The results are illustrated in FIGS. 22A and 22B.

As illustrated in FIGS. 22A and 22B, with respect to the choroidal neovascular lesion, a significantly decreased retinal thickness (220.6±10.47 microns) was observed in the Nkx3.2-overexpressing mice (G2) as compared with that (259.8±13.08 microns) in the wild-type mice (G1) (P<0.01). From these results, it was found that the retinal edema, which occurred secondarily due to the blood retinal barrier destroyed in the process of inducing choroidal neovascularization, was significantly decreased in the Nkx3.2-overexpressing mice as compared with the wild-type mice.

## Claims

1. A pharmaceutical composition for use in preventing or treating retinal dystrophies, comprising as an active ingredient:
Nkx3.2 or a fragment thereof,
wherein the Nkx3.2 or fragment thereof is a polypeptide consisting of the amino acid sequence of SEQ ID NO: 7 or 12 respectively.

2. The pharmaceutical composition for use according to claim 1, wherein the retinal dystrophy is any one selected from the group consisting of macular degeneration, diabetic retinopathy, choroidal neovascularization, and retinal edema.

3. The pharmaceutical composition for use according to claim 2, wherein the macular degeneration is dry macular degeneration or wet macular degeneration.

4. A pharmaceutical composition for use in preventing or treating retinal dystrophies, comprising, as an active ingredient, a recombinant virus that contains a polynucleotide encoding Nkx3.2 or a fragment thereof,
wherein the Nkx3.2 or fragment thereof is a polypeptide consisting of the amino acid sequence of SEQ ID NO: 7 or 12 respectively.

5. The pharmaceutical composition for use according to claim 4, wherein the virus is any one selected from the group consisting of adenovirus, adeno-associated virus (AAV), retrovirus, lentivirus, herpes simplex virus, and vaccinia virus.

6. The pharmaceutical composition for use according to claim 4 or 5, wherein the retinal dystrophy is any one selected from the group consisting of macular degeneration, diabetic retinopathy, choroidal neovascularization, and retinal edema.

7. The pharmaceutical composition for use of claim 6, wherein the macular degeneration is dry macular degeneration or wet macular degeneration.

## Patentansprüche

1. Pharmazeutische Zusammensetzung zur Verwendung bei der Vorbeugung oder Behandlung von Netzhautdystrophien, die als Wirkstoff Folgendes umfasst:
Nkx3.2 oder ein Fragment davon,
wobei das Nkx3.2 oder ein Fragment davon ein Polypeptid ist, das aus der Aminosäuresequenz von SEQ ID NO: 7 bzw. 12 besteht.

2. Pharmazeutische Zusammensetzung zur Verwendung nach Anspruch 1, wobei es sich bei der Netzhautdystrophie um eine beliebige Erkrankung aus der Gruppe handelt, die aus Makuladegeneration, diabetischer Retinopathie, choroidaler Neovaskularisation und Netzhautödem besteht.

3. Pharmazeutische Zusammensetzung zur Verwendung nach Anspruch 2, wobei es sich bei der Makuladegeneration um eine trockene Makuladegeneration oder eine feuchte Makuladegeneration handelt.

4. Pharmazeutische Zusammensetzung zur Verwendung bei der Vorbeugung oder Behandlung von Netzhautdystrophien, die als Wirkstoff ein rekombinantes Virus umfasst, das ein Polynukleotid enthält, das für Nkx3.2 oder ein Fragment davon kodiert,
wobei das Nkx3.2 oder ein Fragment davon ein Polypeptid ist, das aus der Aminosäuresequenz von SEQ ID NO: 7 bzw. 12 besteht.

5. Pharmazeutische Zusammensetzung zur Verwendung nach Anspruch 4, wobei das Virus ein beliebiger ist, ausgewählt aus der Gruppe, die aus Adenovirus, adenoassoziiertem Virus (AAV), Retrovirus, Lentivirus, Herpes-simplex-Virus und Vacciniavirus besteht.

6. Pharmazeutische Zusammensetzung zur Verwendung nach Anspruch 4 oder 5, wobei es sich bei der Netzhautdystrophie um eine beliebige Erkrankung aus der Gruppe handelt, die aus Makuladegeneration, diabetischer Retinopathie, choroidaler Neovaskularisation und Netzhautödem besteht.

7. Pharmazeutische Zusammensetzung zur Verwendung nach Anspruch 6, wobei es sich bei der Makuladegeneration um eine trockene Makuladegeneration oder eine feuchte Makuladegeneration handelt.

## Revendications

1. Composition pharmaceutique destinée à être utilisée dans la prévention ou le traitement des dystrophies rétiniennes, comprenant en tant que principe actif :
du Nkx3.2 ou un fragment de celui-ci,
dans laquelle le Nkx3.2 ou le fragment de celui-ci est un polypeptide consistant en la séquence d'acides aminés de SEQ ID NO : 7 ou 12 respectivement.

2. Composition pharmaceutique destinée à être utilisée selon la revendication 1, dans laquelle la dystrophie rétinienne est l'une quelconque choisie dans le groupe consistant en dégénérescence maculaire, rétinopathie diabétique, néovascularisation choroïdienne, et œdème rétinien.

3. Composition pharmaceutique destinée à être utilisée selon la revendication 2, dans laquelle la dégénérescence maculaire est une dégénérescence maculaire sèche ou une dégénérescence maculaire humide.

4. Composition pharmaceutique destinée à être utilisée dans la prévention ou le traitement des dystrophies rétiniennes, comprenant, en tant que principe actif, un virus recombinant qui contient un polynucléotide codant le Nkx3.2 ou un fragment de celui-ci,
dans laquelle le Nkx3.2 ou le fragment de celui-ci est un polypeptide consistant en la séquence d'acides aminés de SEQ ID NO : 7 ou 12 respectivement.

5. Composition pharmaceutique destinée à être utilisée selon la revendication 4, dans laquelle le virus est l'un quelconque choisi dans le groupe consistant en adénovirus, virus adéno-associé (AAV), rétrovirus, lentivirus, virus de l'herpès simplex et virus de la vaccine.

6. Composition pharmaceutique destinée à être utilisée selon la revendication 4 ou la revendication 5, dans laquelle la dystrophie rétinienne est l'une quelconque choisie dans le groupe consistant en dégénérescence maculaire, rétinopathie diabétique, néovascularisation choroïdienne, et œdème rétinien.

7. Composition pharmaceutique destinée à être utilisée selon la revendication 6, dans laquelle la dégénérescence maculaire est une dégénérescence maculaire sèche ou une dégénérescence maculaire humide.
